# EUROPEAN PATENT SPECIFICATION

(11) **EP 0 403 699 B1**
(45) Date of publication and mention of the grant of the patent: **07.09.1994**
(21) Application number: 89306185.3
(22) Date of filing: 19.06.1989
(51) Int. Cl.: G01N 33/24

(54) **Method and apparatus for measurement of in-situ horizontal stress of non-coherent soil**
Methode und Apparat zur Vor Ort-Messung der horizontalen Spannung im losen Boden
Méthode et appareil de mesure des contraintes in-situ de sols non cohérents

(43) Date of publication of application: 27.12.1990
(73) Proprietor: TAKENAKA CORPORATION, Osaka 541 (JP); TOKYO SOIL RESEARCH CO. LTD, Tokyo (JP)
(72) Inventor: Suzuki, Yoshio, Tokyo (JP); Hatanaka, Munenori, Tokyo (JP); Ohara, Junryou, Tokyo (JP); Makihara, Yorio, Tokyo (JP)
(74) Representative: Clifford, Frederick Alan

(56) References cited:
- WO-A-79/01099
- GB-A- 1 294 062
- US-A- 4 753 107

## Description

The present invention relates to a laboratory test method and apparatus, and a field test method and apparatus, for measuring the in-situ horizontal stress of non-coherent soil such as sand and gravel.

Measurement of the in-situ stress in soil is of major importance in a wide variety of geotechnical problems, but is difficult to carry out accurately and reproducibly in the case of non-coherent soil deposits such as sand, gravel or like loose particulate material.

GB-A-1,294,062 discloses a ground strain gauge comprising a chamber with flexible side walls; a measuring cylinder connected to the chamber; a closed storage tank disposed above the level of the measuring cylinder, the top and the bottom of storage tank being connected to the top part of the measuring cylinder shut-off valves are mounted at the inlet and the outlet side of the measuring cylinder, and a connection on the storage tank for a pressurized gas source.

US-A-4,753,107 discloses a core holder for use in the testing of a geologic core sample. An elastic rubber sleeve for receipt of a core sample is disposed in an open-ended, cylindrical cavity of an open-ended, cylindrical body. The sleeve is compressed by pressurized fluid to apply radial force to the core sample.

WO-A-7 901 099 discloses device for measuring pore water pressure in soils employing a pore pressure sound as a sensing member and a closed measuring system for creating the least possible disturbance in soils having a low degree of permeability, such as clay. The closed liquid-filled measuring system comprises a hypodermic needle type of connection between the pore pressure sound and a measuring device. The needle is operative to penetrate a member of resilient material to extend into a liquid-filled chamber in the pore pressure sound and transfer a reading of the pore water pressure to the measuring device.

The present invention sets out to make such measurement by studying the thawing of a frozen core sample, or of the walls of a borehole extending within a frozen soil volume.

In one form the invention sets out to provide a laboratory test method for determining the in situ lateral stress of non-coherent soil in which a high quality core sample is removed from a frozen volume of the soil at the required location and permitted to thaw within a pressurised test chamber under pressure conditions such as to retain its dimension, the exerted pressure being a measure of the lateral stress; and sets out moreover to provide suitable apparatus for this method.

In another form the invention sets out to provide a field test method for determining the in situ lateral stress of non-coherent soil, in which a high quality core sample is removed from a bore to leave a high quality bore within a frozen volume of the soil, and the walls of this bore are permitted to thaw about a pressurised cylindrical test member under pressure condition such that the walls retain their shape, the exerted pressure being a measure of the lateral stress; and sets out moreover to provide suitable apparatus for this method.

In one aspect the invention consists in a laboratory test method for measuring the in-situ lateral stress of non-coherent soil comprising:
(a) freezing a volume of soil in situ at a predetermined depth
(b) coring the frozen volume to provide a test sample
(c) surrounding the sample with a rubber membrane
(d) enclosing the membrane with a cell to which liquid is admitted to surround the membrane
(e) applying to the specimen a vertical load equivalent to the effective vertical stress at the core depth
(f) allowing the frozen specimen to thaw
(g) adjusting the pressure of surrounding liquid to maintain liquid level constant despite radial strain exerted during thawing, and
(h) noting the internal cell pressure when the sample is thawed as a measure of the in-situ lateral stress in the non-coherent soil.

In another aspect the invention consists in equipment for measuring in situ lateral stress of non-coherent soil, by the above method, comprising:
(a) an outermost cell housing
(b) means for holding a membrane-covered frozen core specimen within the cell
(c) means for exerting pressure on the specimen in an axial direction to reproduce the effective vertical stress at the core depth
(d) air supply means to the cell
(e) an inner cell construction surrounding the specimen location
(f) a float for floating on the surface of liquid in the inner cell
(g) sensor means to detect the vertical position of the float, and
(h) means responsive to the float position for controlling air supply to the air supply means:

whereby the cell and inner cell can be filled with liquid to a level above that of specimen and, as the specimen thaws, be held at their original level by sensor-controlled supply of pressurised air so as to restrain lateral displacement of the specimen, the inner cell air pressure being a measure of the in-situ lateral stress of the non-coherent soil.

In a further and related aspect the invention consists in a field test method for measuring the in situ lateral stress of non-coherent soil, comprising:
(a) freezing a volume of the soil in-situ at a predetermined depth
(b) boring a hole in the frozen soil,
(c) lowering into the hole a test apparatus surrounded by a rubber membrane
(d) inflating the membrane into conformity with the walls of the hole by admitting water to the test apparatus
(e) adjusting the pressure exerted by the water at a membrane to compensate for displacement due to thawing of the walls of the hole
(f) noting the said pressure when thewalls are thawed as a measure of the in-situ lateral stress in the non-coherent soil.

In a yet further aspect the invention consists in field equipment for measuring in-situ lateral stress of non-coherent soil, using the method of the preceding paragraph, comprising
(a) a cylindrical test member;
(b) a rubber membrane speced from the surface of the test member to define an inflatable gap and the constitute an outer surface
(c) water supply means for the inflatable gap
(d) heater means within the inflatable gap for heat supply to water held therein
(g) air-pressure-exerting means for said water supply and
(f) means for determining air pressure:
   whereby, when the test member is located in a bore formed in an in-situ frozen volume in the soil, and the membrane conformed with the bore walls, the exerted air pressure to prevent lateral displacement of the walls of the bore as the frozen material thaws is a measure of the in-situ lateral stress of the non-coherent soil.

Preferably, a pore water pressure transducer is fixed to the apparatus, e.g. at the base, so that the pressure due to water in the pores of the non-coherent soil can be separately measured and distinguished from the lateral stresses of the soil itself.

The method and equipment for measuring the in-situ lateral stress of non-coherent soil according to the present invention will become more apparent from the following description taken in conjuction with the accompanying drawings, in which:
Fig.1 shows diagrammatically in-situ ground freezing of moist non-coherent soil and coring of the frozen ground,
Fig.2 is cross sectional diagram of the test apparatus used in the laboratory test method of measuring the in-situ lateral stress of non-coherent soil by thawing of a frozen test specimen prepared from a high quality undisturbed sample cored from the ground frozen in-situ as described in relation to Figure 1,
Fig.3 shows the essentials of a field test to measure in-situ horizontal (lateral) stress of the non-coherent soil, and
Fig.4 is a cross section of the test apparatus used in the field test.

To obtain a test sample a borehole is first drilled in the ground 1 to about the desired sample depth 11. A freezing pipe 2 (e.g. of about 76 mm external diameter) is placed in the borehole and a coolant such as liquid nitrogen, or ethanol and crushed dry ice mixed with brine, is pumped down the pipe 2. The coolant causes a volume 3 of soil, shown shaded, to freeze and thus cohere. The freezing speed can be controlled by reference to thermocouples (not shown) in the soil. A typical overall lateral dimension of the frozen volume, if gravel, is about 120 cm, and if sand is about 50 cm.

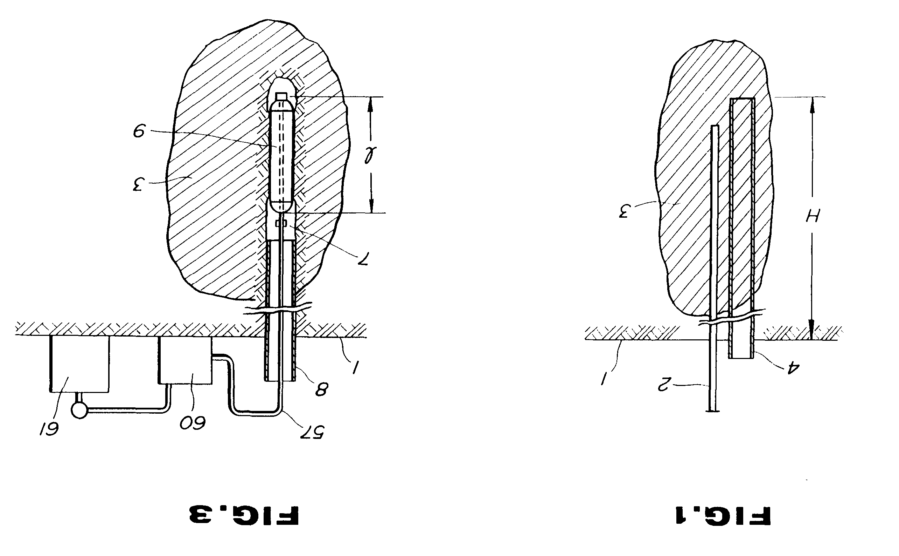

At this stage there are alternative ways of proceeding. One method is to remove essentially the whole volume 3 of frozen soil in a large diameter steel pipe drilled down to a suitable extent and then to core samples from this removed and stored block. The other, as shown in Figure 1 of the drawing, is to core a small-diameter sample directly from a convenient region of the innermost frozen volume 3. In each case, to get a good sample, the regions nearest the freezing pipe 2 or nearest the surface of the frozen volume 3 should be avoided. A typical gravel sample is say 30 cm in diameter: a typical sand sample is 5-10 cm in diameter.

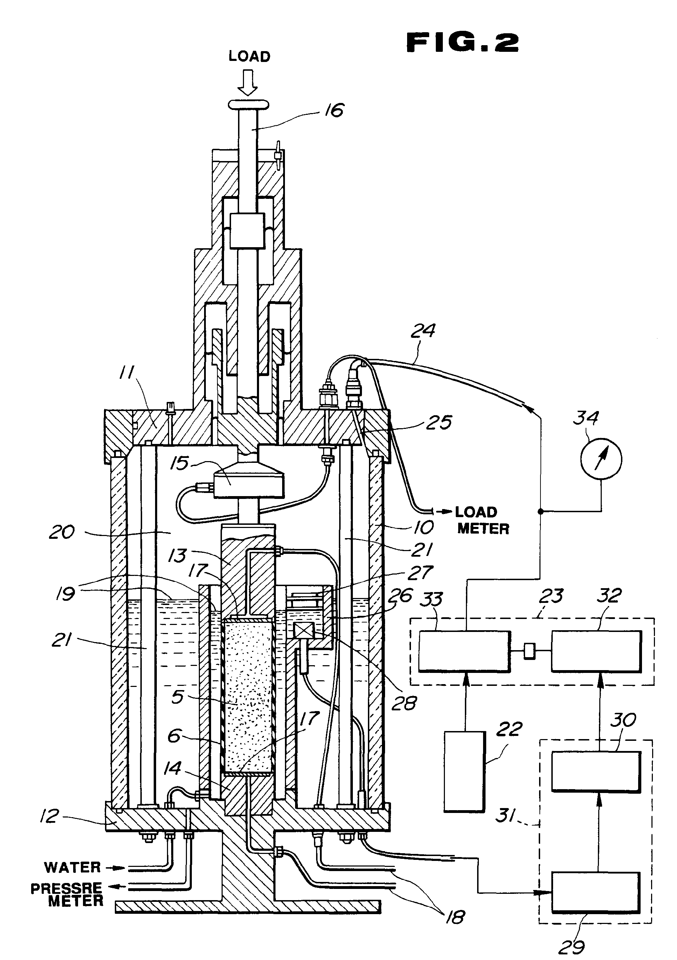

Fig.2 is a cross section of laboratory test apparatus for measuring the in-situ horizontal stress of non-coherent soil. In this apparatus a frozen test specimen obtained as described above was evaluated.

A pressure cell consists of a transparent plastic cylinder 10 and top plate 11 and base plate 12. Top plate 11 and base plate 12 are connected tightly by three stainless steel bars 21. The frozen test specimen 5 is covered with an impermeable rubber membrane 6 sealed to a pedestal 14 and to top a cap 13.

The top cap 13 is connected to a loading rod 16 and axial load transducer 15. The pedestal 14 is fixed on the base plate 12. There is a porous stone installed on the surface of the top cap 13 and pedestal 14 at the side faced to the test specimen. The top cap 13 and the pedestal 14 are connected to the drain pipe 18. The cell is filled with water 19 to a level somewhat higher than the top surface of the test specimen. The space 20 above the water 19 in the cell is filled with air. The air pressure is supplied from the compressor 22 as adjusted by regulator 23 and applied to the space 20 through plastic tube 24 which is connected to the inlet 25 in the top plate 11.

An inner cell 26 is fixed on the base plate 12 in the Pressure cell. The float 27 is placed on the surface of the water 19 inside the inner cell 26. The gap sensor 28 for measuring the vertical movement of the water level in the inner cell 26 is fixed just below the float 27 to the inner cell 26. The vertical movement of the level of the water 19 can be indicated by the vertical displacement of the float 27, which will induce voltage change in the gap sensor 28. The induced voltage is amplified by amplifier 29 and is input to the controller 31 which consists of servo controller 30, servo motor 32 and regulator 33 for adjusting the air pressure to keep the level of the water 19 constant. The lateral strain of the test specimen 5 exerted during thawing is then restrained. The air pressure in the inner cell (measured by pressure transducer 34) when the frozen test specimen is completely thawed indicates the lateral stress of the soil in-situ.

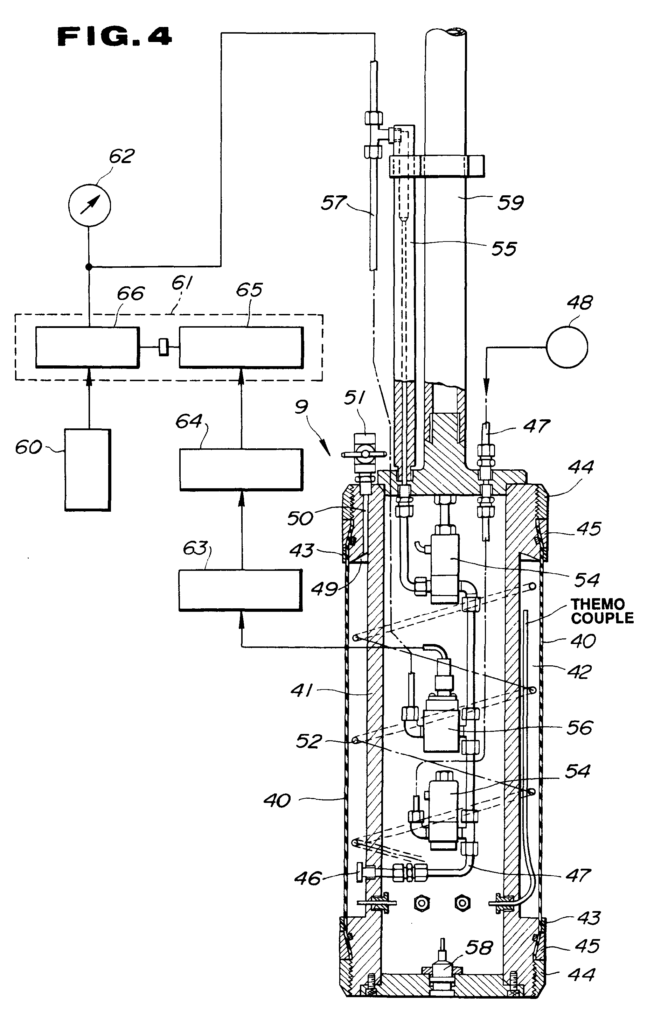

Fig.3 shows an arrangement for effecting a field test method to measure the in-situ horizontal stress of non-coherent soil is performed within a bored hole 7 prepared for instance by coring the ground as frozen by in-situ freezing to obtain a high quality undisturbed sample for the laboratory test described above, and as described in relation to Fig. 1.

A steel pipe 8 as inserted into the bore hole 7 to a depth somewhat shallower than the depth for testing this keeps the inside surface of the hole from collapse. The test apparatus 9, about 60 cm in length, having a rubber balloon-like structure about its surface is installed in the bored hole at the test depth. Suitable air pressure is applied to ensure that this rubber balloon fits to the wall surface of the frozen bored hole 7.

Fig.4 is a cross section of the test apparatus 9. The rubber membrane or balloon (40) is e.g. about 40 cm in length and of almost the same diameter as the bored hole. It is fixed to both ends of the cylindrical shape housing 41 by a steel ring 44 associated with steel ring 45 and a back-up ring 43 made of rubber. Thus, closed space 42 is sealed off as a balloon. The space 42 is filled with deaerated water through the pipe 47 connected to the inlet 46 inside of the housing 41. The pipe 47 for supplying water is connected to water pump 48. The space 42 is tapered in the upper part 49 in order to ensure that the space 42 is completely filled. Initial air can be easily expressed from the tapered upper part 49 through drilled hole 50 and air valve 51. A coil heater 52 in the space 42 keeps the temperature of the water in the space 42 constant. A water head pipe 55 is connected to a two-way electro-magnetic valve 54 located at the upper part of the inside of the housing 41. The upper part of the water head pipe 55 and differential pressure transducer 56 are connected to the same air pressure supply pipe 57. A pore water pressure transducer 58 is mounted at the base of the housing 41.

The test apparatus 9 is attached to the lower end of a rod and installed at the desired depth for testing as in Fig. 3. The air pressure from the source 60 is adjusted by servo motor 61 and supplied to the pipe 47 through the pipe 57. The air pressure can be monitored from the pressure meter 62. The pressure regulator 66 was operated by servo motor 65 and servo motor regulating valve 61 according to the input from servo controller 64. The input electrical signal to the servo controller 64 is transmitted from the differential pressure measured from the pressure transducer 58 is used for determination of the effective lateral stress in-situ which is needed to calculate the coefficient of earth pressure at rest.

On practice, the space 42 was filled with water and the valve 51 on the ground surface was shut. Then the test probe 9 was installed into the frozen bored hole at a depth to be tested. A small pressure was applied to the space 42 to conform the rubber membrane 40 to the inside surface of the frozen bored hole. The radial displacement of the bored hole 7 during thawing will cause the water level in the water head pipe 55 to rise. The movement of the water level in the pipe 55 will be detected by differential pressure transducer 56, and the regulator 61 will then adjust the air pressure to maintain the level of the water in the pipe 55 constant, which means the no lateral strain in the soil.

The pressure in the space 42, as monitored from the pressure meter 62 at the stage where the frozen bore 3 was completely thawed, indicates the in-situ lateral stress in the soil. The true lateral effective stress in-situ can be calculated from the above monitored air pressure and pore water pressure measured by pressure transducer 58.

The Young's modulus of non-coherent soil in-situ in the horizontal direction can be measured using the test apparatus as used in the field test.

## Claims

1. A laboratory test method for measuring the in-situ lateral stress of non-coherent soil comprising:
(a) freezing a volume (3) of soil in situ at a predetermined depth (11);
(b) coring the frozen volume to provide a test sample;
(c) surrounding the sample with a rubber membrane (6);
(d) enclosing the membrane with a cell (26) to which liquid (19) is admitted to surround the membrane (6);
(e) applying to the specimen a vertical load equivalent to the effective vertical stress at the core depth;
(f) allowing the frozen specimen to thaw;
(g) adjusting the pressure (30, 32, 33) of surrounding liquid (19) to maintain liquid level constant despite radial strain exerted during thawing; and
(h) noting the internal cell pressure when the sample is thawed as a measure of the in-situ lateral stress in the non-coherent soil.

2. Equipment for measuring in-situ lateral stress of non-coherent soil by the method of claim 1, comprising:
(a) an outermost cell housing (10, 11, 12);
(b) means for holding a membrane-covered frozen core specimen (5) within the cell;
(c) means for exerting pressure on the specimen in an axial direction to reproduce the effective vertical stress at the core depth;
(d) air supply means (22) to the cell
(e) an inner cell construction (26) surrounding the specimen location;
(f) a float (27) for floating on the surface of liquid (19) in the inner cell (26);
(g) sensor means (28) to detect the vertical position of the float, and
(h) means (30, 32, 33) responsive to the float position for controlling air supply to the air supply means:
whereby the cell and inner cell can be filled with liquid (19) to a level above that of specimen and, as the specimen thaws, be held at their original level by sensor-controlled supply of pressurised air so as to restrain lateral displacement of the specimen, the inner cell air pressure being a measure of the in-situ lateral stress of the non-coherent soil.

3. A field test method for measuring the in situ lateral stress of non-coherent soil, comprising:
(a) freezing a volume of the soil in-situ at a predetermined depth;
(b) boring a hole (7) in the frozen soil;
(c) lowering into the hole a test apparatus (9) surrounded by a rubber membrane (40);
(d) inflating the membrane (40) into conformity with the walls of the hole by admitting water to the test apparatus;
(e) adjusting the pressure exerted by the water at a membrane to compensate for displacement due to thawing of the walls of the hole; and
(f) noting the said pressure when the walls are thawed as a measure of the in-situ lateral stress in the non-coherent soil.

4. Equipment for measuring in-situ lateral stress of non-coherent soil by the method of claim 3, comprising:
(a) a cylindrical test member (9);
(b) a rubber membrane (40) spaced from the surface of the test member to define an inflatable gap (42) and to constitute an outer surface;
(c) water supply means (47) or the inflatable gap;
(d) heater means (52) within the inflatable gap for heat supply to water held therein;
(g) air-pressure-exerting means (61) for said water supply; and
(f) means (62) for determining air pressure:
whereby, when the test member is located in a bore formed in an in-situ frozen volume in the soil, and the membrane conformed with the bore walls, the exerted air pressure to prevent lateral displacement of the walls of the bore as the frozen material thaws is a measure of the in-situ lateral stress of the non-coherent soil.

5. Equipment as claimed in claim 4 further comprising a pore water pressure transducer (58) to measure separately the pressure of the water in the pores of the non-coherent soil whereby this value can be distinguished from the measurement of lateral stress.

## Patentansprüche

1. Laborprüfverfahren zur Messung der seitlichen Belastung von losem Boden vor Ort, welches folgende Schritte aufweist:
(a) Gefrieren einer Bodenmenge (3) vor Ort in einer festgelegten Tiefe (11);
(b) Ziehen eines Bohrkerns der gefrorenen Bodenmenge, um eine Prüfprobe zu erhalten;
(c) Umhüllen der Probe mit einer Gummimembran (6);
(d) Einschlließen der Membran durch eine Zelle (26), in die eine Flüssigkeit (19) eingeführt wird, um die Membran (6) zu umhüllen;
(e) Ausübung einer senkrechten Last, die gleich der wirksamen senkrechten Belastung in Kerntiefe ist, auf die Probe;
(f) Auftauenlassen der gefrorenen Probe;
(g) Regulierung des Drucks (30, 32, 33) der umhüllenden Flüssigkeit (19), um den Pegel der Flüssigkeit trotz der radialen Beanspruchung, die während des Tauens wirksam wird, konstant zu halten; und
(h) Notieren des Zell-Innendrucks, wenn die Probe aufgetaut ist, als Maß der seitlichen Belastung vor Ort, die in losem Boden wirksam ist.

2. Apparat zur Messung der seitlichen Belastung von losem Boden vor Ort nach dem Verfahren gemäß Anspruch 1, welcher folgende Elemente aufweist:
(a) ein äußeres Zellgehäuse (10, 11, 12);
(b) Mittel zum Halten einer mit einer Membran bedeckten, gefrorenen Bohrkern-Probe (5) in der Zelle;
(c) Mittel zum Ausüben von Druck in Axialrichtung auf die Probe, um die wirksame senkrechte Belastung in Kerntiefe zu reproduzieren;
(d) Mittel zur Einführung von Luft (22) in die Zelle;
(e) eine innere Zellenkonstruktion (26), welche den Aufbewahrungsort der Probe umschließt;
(f) einen Schwimmkörper (27) zum Schwimmen auf der Oberfläche der Flüssigkeit (19) in der inneren Zelle (26);
(g) Sensormittel (28) zur Feststellung der senkrechten Position des Schwimmkörpers; und
(h) Mittel (30, 32, 33), die auf die Position des Schwimmkörpers ansprechen, um die Luftzufuhr zum Lufteinführungsmittel zu steuern;
bei welchem die Zelle und die innere Zelle bis zu einem Pegel über dem der Probe mit Flüssigkeit (19) gefüllt werden können und, wenn die Probe taut, dieser durch die sensor-gesteuerte Zufuhr von Druckluft beim ursprünglichen Pegel gehalten werden kann, um die seitliche Verschiebung der Probe einzuschränken, wobei der Luftdruck in der inneren Zelle ein Maß für die seitliche Belastung des losen Bodens vor Ort ist.

3. Feldprüfverfahren zur Messung der seitlichen Belastung von losem Boden vor Ort, welches folgende Schritte aufweist:
(a) Gefrieren einer Bodenmenge vor Ort in einer festgelegten Tiefe;
(b) Bohren eines Lochs (7) in den gefrorenen Boden;
(c) Absenken eines Prüfapparates (9), der von einer Gummimembran (40) umschlossen ist, in das Loch;
(d) Aufblähen der Membran (40) zur Angleichung an die Wände des Lochs durch Einführung von Wasser in den Prüfapparat;
(e) Regulierung des Drucks, der durch das Wasser auf die Membran ausgeübt wird, um die Verschiebung aufgrund des Tauens der Wände des Lochs auszugleichen; und
(f) Notieren des Drucks, wenn die Wände aufgetaut sind, als Maß der seitlichen Belastung vor Ort, die in dem losen Boden wirksam ist.

4. Apparat zur Messung der seitlichen Belastung von losem Boden vor Ort nach dem Verfahren gemäß Anspruch 3, welcher folgende Elemente aufweist:
(a) ein zylindrisches Prüfelement (9);
(b) eine Gummimembran (40), die Abstand zur Oberfläche des Prüfelements hat, um eine aufblähbare Lücke (42) zu definieren und um eine Außenfläche zu bilden;
(c) Mittel (47) zur Zuführung von Wasser in die aufblähbare Lücke;
(d) Heizmittel (52) in der aufblähbaren Lücke, um dem darin enthaltenen Wasser Wärme zuzuführen;
(e) Mittel (61) zur Ausübung von Luftdruck auf die Wasserzuführung; und
(f) Mittel (62) zur Bestimmung des Luftdrucks,
bei welchem, wenn sich das Prüfelement in einer Bohrung befindet, die in einer vor Ort gefrorenen Bodenmenge ausgeführt wird, und wenn sich die Membran den Wänden der Bohrung angepaßt hat, der Luftdruck, der ausgeübt wird, um die seitliche Verschiebung der Wände der Bohrung beim Auftauen des gefrorenen Materials zu verhindern, das Maß der seitlichen Belastung des losen Bodens vor Ort ist.

5. Apparat nach Anspruch 4, welcher außerdem einen Druckmeßumformer (58) für das Porenwasser aufweist, um den Druck des Wassers in den Poren des losen Bodens gesondert zu messen, wodurch es möglich ist, diesen Wert vom der Meßwert der seitlichen Belastung zu unterscheiden.

## Revendications

1. Un procédé d'essai en laboratoire pour mesurer la contrainte latérale in situ de sol non cohérent, comprenant les étapes suivantes:
(a) congélation d'un volume (3) de sol in situ à une profondeur prédéterminée (11);
(b) carottage du volume congelé pour produire un échantillon d'essai;
(c) encerclement de l'échantillon d'une membrane en caoutchouc (6);
(d) encadrement de la membrane d'une cellule (26), dans laquelle est admis un liquide (19) pour entourer la membrane (6);
(e) application au spécimen d'une charge verticale équivalente à la contrainte verticale effective au niveau de la profondeur de la carotte;
(f) dégel du spécimen congelé;
(g) ajustement de la pression (30, 32, 33) du liquide entourant (19) pour maintenir le niveau de liquide constant malgré la contrainte radiale exercée au cours du dégel; et
(h) enregistrement de la pression interne de la cellule après le dégel de l'échantillon comme une mesure de la contrainte latérale in situ du sol non cohérent.

2. Equipement pour mesurer la contrainte latérale in situ de sol non cohérent au moyen du procédé selon la revendication 1, comprenant:
(a) un boîtier extérieur de la cellule (10, 11, 12);
(b) un moyen pour maintenir un spécimen de carotte congelé recouvert par une membrane (5) à l'intérieur de la cellule;
(c) un moyen pour exercer une pression sur le spécimen, dans la direction axiale, pour reproduire la contrainte verticale effective au niveau de la profondeur de la carotte;
(d) un moyen d'amenée d'air (22) vers la cellule;
(e) une construction de cellule intérieure (26) entourant l'emplacement du spécimen;
(f) un flotteur (27) pour flotter sur la surface du liquide (19) dans la cellule intérieure (26);
(g) un moyen capteur (28) pour détecter la position verticale du flotteur, et
(h) un moyen (30, 32, 33), réagissant à la position du flotteur pour régler l'amenée d'air vers le moyen d'amenée d'air:
la cellule et la cellule intérieure pouvant ainsi être remplies d'un liquide (19) jusqu'à un niveau au-dessus de celui du spécimen et pouvant, lors du dégel du spécimen, être maintenues au niveau original par une amenée d'air sous pression, réglée par le capteur, de sorte à limiter le déplacement latéral du spécimen, la pression d'air de la cellule intérieure étant une mesure de la contrainte latérale in situ du sol non cohérent.

3. Un procédé d'essai sur le terrain pour la mesure de la contrainte latérale in situ de sol non cohérent, comprenant les étapes suivantes:
(a) congélation d'un volume de sol in situ à une profondeur prédéterminée;
(b) alésage d'un trou (7) dans le sol congelé;
(c) descente d'un dispositif d'essai (9), entouré par une membrane en caoutchouc (40) dans le trou;
(d) expansion de la membrane (40), pour l'adapter aux parois du trou par admission d'eau dans le dispositif d'essai;
(e) ajustement de la pression exercée par l'eau sur une membrane pour compenser le déplacement entraîné par le dégel des parois du trou; et
(f) enregistrement de ladite pression après le dégel des parois comme une mesure de la contrainte latérale in situ dans le sol non cohérent.

4. Equipement pour mesurer la contrainte latérale in situ de sol non cohérent au moyen du procédé selon la revendication 3, comprenant:
(a) un élément d'essai cylindrique (9);
(b) une membrane en caoutchouc (40), espacée de la surface de l'élément d'essai pour définir un espace expansible (42) et pour constituer une surface extérieure;
(c) un moyen d'amenée d'eau (47) dans l'espace expansible;
(d) un moyen de chauffage (52) à l'intérieur de l'espace expansible pour fournir de la chaleur à l'eau qui y est contenue;
(e) un moyen exerçant une pression d'air (61) pour ladite amenée d'eau; et
(f) un moyen (62) pour déterminer la pression d'air:
l'élément d'essai étant agencé dans un alésage formé dans un volume congelé in situ dans le sol et la membrane étant adaptée aux parois de l'alésage, la pression d'air exercée pour empêcher un déplacement latéral des parois de l'alésage lors du dégel du matériau congelé constituant ainsi une mesure de la contrainte latérale in situ du sol non cohérent.

5. Equipement selon la revendication 4, comprenant en outre un transducteur de pression d'eau dans les pores (58) pour mesurer séparément la pression de l'eau dans les pores du sol non cohérent, cette valeur pouvant ainsi être distinguée de la mesure de la contrainte latérale.
